# EUROPEAN PATENT APPLICATION

(11) **EP 4 657 066 A1**
(43) Date of publication of application: **03.12.2025**
(21) Application number: 24178344.8
(22) Date of filing: 28.05.2024
(51) Int. Cl.: G01N 33/24, G01N 27/22

(54) **A SOIL MOISTURE SENSOR**

(71) Applicant: Husqvarna AB, 561 82 Huskvarna (SE)
(72) Inventor: MERKLE, Martin, 89168 NIEDERSTOTZINGEN (DE); ANDRIOF, Frederik, 89171 ILLERKIRCHBERG (DE)
(74) Representative: Finkele, Rolf

(57) **Abstract**

A soil moisture sensor **(100)** comprises a first conductive surface **(210),** a second conductive surface **(220),** and a third conductive surface **(230).** The first conductive surface **(210)** and the second conductive surface **(220)** form a first capacitor **(C1)** configured to generate a primary electric field **(E1),** wherein the primary electric field **(E1)** comprises primary electric field lines **(L1).** The soil moisture sensor **(100)** is characterized in that the soil moisture sensor **(100)** further comprises a fourth conductive surface **(240),** wherein the third conductive surface **(230)** and the fourth conductive surface **(240)** form a second capacitor **(C2)** configured to generate a secondary electric field **(E2),** and wherein the secondary electric field **(E2)** comprises secondary electric field lines **(L2).** Further, the first capacitor **(C1)** and the second capacitor **(C2)** are arranged such that the first capacitor **(C1)** is nested within the second capacitor **(C2).** Further, the primary electric field lines **(L1)** are separated from the secondary electric field lines **(L2)** such that a capacitance measuring of the moisture within the surrounding soil is allowed.

## Description

### TECHNICAL FIELD

The present disclosure relates to a soil moisture sensor, particularly the capacitive soil moisture sensor for measuring a moisture within a soil surrounding the soil moisture sensor.

### BACKGROUND

Water saving is an increasingly important social issue as population increases in urbanized areas, and variable weather patterns create geographic areas of water stress. Measuring soil moisture is important for agricultural applications to help farmers manage their irrigation systems more efficiently. Knowing the exact soil moisture conditions on their fields, not only allows farmers to generally use less water to grow a crop, but they also allows farmers to increase yields and the quality of the crop by improved management of soil moisture during critical plant growth stages. In urban and suburban areas, landscapes and residential lawns are using soil moisture sensors to interface with an irrigation controller. Connecting a soil moisture sensor to a simple irrigation clock converts it into a "smart" irrigation controller that prevents irrigation cycles when the soil is already wet, e.g., following a recent rainfall event. In other situations, golf courses are using soil moisture sensors to increase the efficiency of their irrigation systems to prevent over-watering and leaching of fertilizers and other chemicals into the ground.

Wired soil moisture sensors are known that uses a small thermal measuring tip surrounded by a water-absorbing material, e.g., felt. To determine the moisture, the measuring tip is heated up and subsequently it is measured how long it takes for the tip to reach approximately its initial temperature. Since moisture increases the thermal conductivity, shorter cooling times are measured in the case of high moisture. The disadvantage of this method, apart from its high cost, is that it is very energy-intensive due to the heating process. Due to the relatively slow cooling, it also takes up to two minutes after triggering the measurement until a measured value can be determined. In addition, several measurements cannot be made directly one after the other, because otherwise the measuring tip heats up more and more. This time behavior is particularly disadvantageous since a user cannot always start a measurement himself or since the measured values are not up-to-date because a measurement is not always possible. Further, even if a measurement is possible, the user may not immediately receive a result.

For the reasons mentioned above, a favorable and energy-saving capacitive measuring method is preferred for smart soil moisture sensor. However, this requires a relatively large sensor area, making it difficult to insert the sensor into the ground. In addition, the sensor directly measures volumetric water content, which has very little correlation with plant-available water and is soil-dependent.

An example of a soil moisture sensor is provided in United States Patent US 9,949,450 B2 (hereinafter referred to as '450 reference). The '450 reference provides a system for controlling irrigation and comprising a wireless transmitter tethered to a stand-alone soil moisture probe by a length of cable. The soil moisture probe has first and second elongate conductive materials encapsulated in a non-conductive substrate. The first and second elongate materials are coupled to a power source and are configured to be placed in the soil to form a capacitor. The soil moisture probe also has a soil moisture circuit encapsulated in the non-conductive substrate coupled to the power source such that the circuit has an oscillator for applying an electrical stimulus to the first and second elongate conductive materials. In addition, a third elongate conductive material is encapsulated in the non-conductive substrate and disposed between the first and second elongate conductive materials. The third elongate material is coupled to the soil moisture circuit and is conductively isolated from the first and second elongate conductive materials.

### SUMMARY OF THE INVENTION

In view of the above, it is an objective of the present invention to solve or at least reduce the drawbacks discussed above. The objective is at least partially achieved by a soil moisture sensor.

According to an aspect of the present invention, the soil moisture sensor for measuring a moisture within a soil surrounding the soil moisture sensor comprises a first capacitor configured to generate a primary electric field comprising primary electric field lines. The soil moisture sensor is characterized in that the soil moisture sensor further comprises a second capacitor configured to generate a secondary electric field comprising secondary electric field lines, wherein the first capacitor and the second capacitor are arranged such that the first capacitor is nested within the second capacitor, and wherein the primary electric field lines are separated from the secondary electric field lines such that a capacitance measuring of the moisture within the surrounding soil is enabled.

The present disclosure provides the soil moisture sensor, which is cost-effective, reliable, and efficient. The soil moisture sensor advantageously comprises the first capacitor and the second capacitor such that the first capacitor is nested within the second capacitor to improve the accuracy of the measurements provided by the soil moisture sensor. Further, by nesting the first capacitor within the second capacitor, it is possible to separate the primary electric field lines and the secondary electric field lines. Hence, measuring the difference in capacitance of the surrounding which results in the amount of moisture is possible.

The primary electric field lines are separated from and nested inside of the secondary electric field lines such that the secondary electric field lines are pushed away from the primary electric field. Hence, the secondary electric field lines are pushed further outwards respectively away from the second capacitor and thereby further into the surrounding soil.

According to an exemplary embodiment of the present invention, the first capacitor is formed by a first conductive surface and a second conductive surface, wherein the first conductive surface and the second conductive surface are configured such that the primary electric field is formed between the first conductive surface and the second conductive surface. The second capacitor is formed by a third conductive surface and a fourth conductive surface, wherein the third conductive surface and the fourth conductive surface are configured such that the secondary electric field is formed between the third conductive surface and the fourth conductive surface. The first conductive surface and the third conductive surface are formed as two separate conductive surfaces, and the second conductive surface and the fourth conductive surface are formed as one common conductive surface.

By forming the second conductive surface and the fourth conductive surface as one common conductive surface, the soil moisture sensor may be compact and cost-efficient.

Forming the second conductive surface and the fourth conductive surface as one common conductive surface may denote that one conductive surface is provided/formed which is connected and switched respectively used such that the common conductive surface is at the same time a further conductive surface (here the second conductive surface) of the first capacitor and a further conductive surface (here the fourth conductive surface) of the second capacitor. Hence, the one common conductive surface forms together with the first conductive surface the nested first capacitor, and at the same time with the third conductive surface the surrounding second capacitor.

Forming the first conductive surface and the third conductive surface as two separate conductive surfaces may denote that the first conductive surface and the third conductive surface are two distinguished respectively spaced apart surfaces. Particularly the two conductive surfaces are formed respectively arranged distanced from each other.

According to an exemplary embodiment of the present invention, the first capacitor is formed by a first conductive surface and a second conductive surface, wherein the first conductive surface and the second conductive surface are configured such that the primary electric field is formed between the first conductive surface and the second conductive surface. The second capacitor is formed by a third conductive surface and a fourth conductive surface, wherein the second conductive surface and the fourth conductive surface are configured such that the secondary electric field is formed between the third conductive surface and the fourth conductive surface. The first conductive surface and the third conductive surface are formed as two separate conductive surfaces, and the second conductive surface and the fourth conductive surface are formed as two separate conductive surfaces.

By forming the second conductive surface and the fourth conductive surface as two separated conductive surfaces, the soil moisture sensor may be reliable and may be operated in a fail-safe manner.

Forming the second conductive surface and the fourth conductive surface as two separate conductive surfaces may denote that two distinguished respectively spaced apart conductive surfaces are provided. In other words, the second conductive surface and the fourth conductive surface are formed distanced and spaced apart from each other. Further, the second conductive surface and the fourth conductive surface may be electrically isolated from each other.

Forming the first conductive surface and the third conductive surface as two separate conductive surfaces may denote that the first conductive surface and the third conductive surface are two distinguished surfaces. Specifically, the first conductive surface and the third conductive surface may be formed spaced apart surfaces, particularly formed respectively arranged distanced from each other. Alternatively, the first conductive surface and the third conductive surface may be separated from each, particularly electrically separated from each other, e.g., by applying a thin isolating layer between the two surfaces.

According to an exemplary embodiment of the invention, the primary electric field is a parasitic field, and/or the secondary electric field is a measuring field. The first capacitor is configured to generate the parasitic field which may be separated or decoupled from the measuring field which is generated by the second capacitor. Thereby, an accuracy and/or sensitivity in the moisture measurement is increasable.

According to an exemplary embodiment of the invention, the first capacitor and the second capacitor are simultaneously excited with a same signal. The first capacitor and the second capacitor may be excited with the same signal, and at the same time. This results in no potential difference between the third conductive surface and the first conductive surface, respectively between the fourth conductive surface and the second conductive surface. Further, the first capacitor and the second capacitor do substantially not influence each other to allow clean separation between the parasitic field and the measuring field.

According to an exemplary embodiment of the invention, the first capacitor and the second capacitor are excited with a 10kHz sinusoidal signal. The first capacitor and the second capacitor may be excited with the 10kHz sinusoidal signal simultaneously. This results in no potential difference between the third conductive surface and the first conductive surface respectively between the second conductive surface and the fourth conductive surface. Further, the first capacitor and the second capacitor do substantially not influence each other to allow separation between the parasitic field and the measuring field of the first capacitor and the second capacitor respectively.

According to an exemplary embodiment of the invention, the capacitance of the moisture within the surrounding soil is measured from the resulting current. The first capacitor and the second capacitor may be excited with the same signal. Further, the capacitance/humidity may be determined from the resulting current. In addition, the phase shift between the excitation and the resulting current may be used to infer a parallel conductance to the capacitor, which is caused by the conductivity of the earth.

According to an exemplary embodiment of the invention, the third conductive surface is arranged adjacent to the first conductive surface, and/or the first conductive surface is arranged in-between the third conductive surface and the second conductive surface. The first conductive surface, the second conductive surface, the third conductive surface, and the fourth conductive surface may be arranged in a manner such that the first conductive surface and the second conductive surface are nested within respectively between the third conductive surface and the fourth conductive surface. This arrangement of conductive surfaces may guide the outer respectively surrounding electrical field outwards. Hence, a measurement accuracy and sensitivity in the surrounding area in which an amount of moisture is measurable by the soil moisture sensor may be increased.

According to an exemplary embodiment of the invention, the fourth conductive surface is arranged adjacent to the second conductive surface, and/or the second conductive surface is arranged in-between the first conductive surface and the fourth conductive surface The first conductive surface, the second conductive surface, the third conductive surface, and the fourth conductive surface may be arranged in a manner such that the first conductive surface and the second conductive surface are nested within respectively between the third conductive surface and the fourth conductive surface. This arrangement of conductive surfaces may reliably separate the parasitic field generated by the first capacitor from the measuring field generated by the second capacitor. Hence, the amount of moisture may be measured more accurate.

According to an exemplary embodiment of the invention, the first conductive surface comprises a first width extending perpendicular to a first length of the first conductive surface. Further, the second conductive surface comprises a second width extending perpendicular to a second length of the second conductive surface. Further, the third conductive surface comprises a third width extending perpendicular to a third length of the third conductive surface. Further, the fourth conductive surface comprises a fourth width extending perpendicular to a fourth length of the fourth conductive surface. The structure and dimensions of the four conductive surfaces may be set such that efficient working of the soil moisture sensor is allowable.

According to an exemplary embodiment of the invention, the first width and the second width are substantially equal, and/or the third width and the fourth width are substantially equal, and/or the first length is substantially equal to the second length, and/or the third length is substantially equal to the fourth length. Hence, a surface area and shape of the first conductive surface is substantially equal to a surface area and shape of the second conductive surface. Additionally or alternatively, a surface area and shape of the third conductive surface is substantially equal to a surface area and shape of the fourth conductive surface. Thereby, a distribution of the primary electrical field lines and/or the secondary electrical field lines is more uniform. Hence, a uniformity of the primary electrical field and/or the secondary electrical field is/are more balanced.

According to an exemplary embodiment of the invention, the first length is greater than or substantially equal to the third length, and/or the second length is greater than or substantially equal to the fourth length.

When the first length is substantially equal to the third length and/or the second length is substantially equal to the fourth length, the secondary electric field lines are forced respectively pushed outwards respectively away from a tip region of the second capacitor. Thereby, the secondary electric field respectively the measuring field is pushed outwards from the tip region. This results in an increased measuring area of the soil moisture sensor.

When the first length is greater than the third length and/or the second length is greater than the fourth length, the secondary electrical field lines are forced respectively pushed even further outwards respectively away from a tip region of the second capacitor. Thereby, the secondary electrical field respectively the measuring field is pushed further outwards. Hence, the measuring area may be increased.

According to an exemplary embodiment of the invention, the first width is greater than or substantially equal to the third width, and/or the second width is greater than or substantially equal to the fourth width.

When the first width is substantially equal to the third width and/or the second width is substantially equal to the fourth width, the secondary electric field lines are forced respectively pushed outward/away from lateral edge areas of the second capacitor. Thereby, the secondary electric field respectively the measuring field is pushed outwards/away from lateral edge regions of the soil moisture sensor. This results in an increased measuring area of the soil moisture sensor.

When the first width is greater than the third width and/or the second width is greater than the fourth width, the secondary electrical field lines are forced respectively pushed even further outwards respectively away from lateral edge areas of the second capacitor. Thereby, the secondary electrical field respectively the measuring field is pushed even further outward. This results in an increased measuring area of the soil moisture sensor. Thereby, the measuring area of the soil moisture sensor may be even more increased.

The greater width or length of one of the two adjacent conductive surfaces may be caused by physical enlargement of the respective conductive surface. Alternatively, a part of one conductive surface may be divided by switching or energizing the part correspondingly to the other conductive surface.

According to an exemplary embodiment of the invention, the soil moisture sensor is at least partially surrounded by a water-absorbing material. The water-absorbing material may completely surround the various conductive surfaces of the soil moisture sensor. Further, the water-absorbing material soak up the plant available water present in the soil surrounding the soil moisture sensor, to facilitate and increase the accuracy of the moisture measuring .

According to an exemplary embodiment of the invention, the soil moisture sensor is wirelessly connected with an irrigation system to control irrigation. The soil moisture sensor may be wirelessly or via wire connected to the irrigation system to govern the working of the irrigation system based on the output of the soil moisture sensor. For example, the irrigation system may be a watering computer and based on the output of the soil moisture sensor may not initiate watering, if the soil is already moist. A wireless connection may increase the areas of application of the soil moisture sensor.

According to an exemplary embodiment of the invention, the first conductive surface, the second conductive surface, the third conductive surface, and the fourth conductive surface have a width ranging from 1.5 mm to 5.5 mm, in particular from 3 mm to 4 mm, more in particular substantially 3.5 mm. According to an exemplary embodiment of the invention, the first conductive surface, the second conductive surface, the third conductive surface, and the fourth conductive surface have a length ranging from 5 mm to 20 mm, in particular 10 mm to 15 mm, more in particular 12 mm. These dimensions may provide a combination of a compact overall size of the soil moisture sensor and a sufficient large area of moisture measuring around the soil moisture sensor.

Further, the secondary electric field lines may be long field lines which are less sensitive to air gaps and even allow a complete waterproof encapsulation of the soil moisture sensor from the surrounding medium.

Before discussing the invention with the help of the drawings, the invention will be briefly discussed in general. A soil moisture sensor is provided with a four-layer PCB with two pairs of capacitors directly opposite each other. The inner two layers, i.e., a first conductive surface and a second conductive surface, form a first capacitor and cover the direct field through the base material. In common sensor setups, it is exactly this parasitic capacitor that contributes a constant offset to the measured capacitance but is not dependent on humidity. By using the two outer layers, i.e., a third conductive surface and a fourth conductive surface, the described parasitic field can be decoupled from the much smaller stray field. This makes it possible to determine the humidity of the environment with the actual stray field. In addition, due to its long field lines, it is less sensitive to air gaps and even allows a complete waterproof encapsulation of the sensor from the surrounding medium.

Further, according to an exemplary embodiment, both capacitors are excited with the same signal and at the same time. Hence, there is no potential difference between the conductive surfaces on one side (e.g., there is no potential difference between the first conductive surface and the third conductive surface on the one side and between the second conductive surface and the fourth conductive surface on the other side) and the capacitors hardly influence each other, which allows the clear separation between the parasitic field and the measuring field respectively stray field. The capacitors can be excited with a 10kHz sinusoidal signal, for example, and the capacitance/humidity can be determined from the resulting current. In addition, the phase shift between the excitation and the resulting current can be used to infer a parallel conductance to the capacitor. This can be caused either by the conductivity (mineral content) of the earth or, in the event of a fault, by moisture in the measuring system.

The soil moisture sensor according to the present invention may be cost-effective, energy saving, with no time delay (< 1 sec). The soil moisture sensor according to the present invention may be completely surrounded by a housing. The soil moisture sensor according to the present invention may measure plant available water rather than pure volumetric water content. The soil moisture sensor according to the present invention may be able to detect a malfunction respectively a fault.

According to an exemplary embodiment of the invention, the soil moisture sensor may separate respectively decouple the parasitic field and the measuring field. According to an exemplary embodiment of the invention, the soil moisture sensor may involve simultaneously excitation of both capacitors and current measurement (amplitude + phase + RMS) for evaluation. According to an exemplary embodiment of the invention, the soil moisture sensor, particularly the capacitive soil moisture sensor, may be completely surrounded by a water-absorbing material.

Other features and aspects of this invention will be apparent from the following description and the accompanying drawings.

### BRIEF DESCRIPTION OF THE DRAWINGS

The invention will be described in more detail with reference to the enclosed drawings, wherein:
**FIG. 1** illustrates a schematic view of a soil moisture sensor in accordance with an exemplary embodiment of the present disclosure;
**FIG. 2** illustrates an exploded schematic view of a soil moisture sensor in accordance with an exemplary embodiment of the present disclosure;
**FIG. 3** illustrates a schematic view of a first capacitor and a second capacitor in accordance with an exemplary embodiment of the present disclosure; and
**FIG. 4** illustrates a schematic view of a soil moisture sensor surrounded by a water-absorbing material in accordance with an exemplary embodiment of the present disclosure.

### DETAILED DESCRIPTION OF THE DRAWINGS

The present invention will be described more fully hereinafter with reference to the accompanying drawings, in which example embodiments of the invention incorporating one or more aspects of the present invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. For example, one or more aspects of the present invention may be utilized in other embodiments and even other types of structures and/or methods. In the drawings, like numbers refer to like elements.

Certain terminology is used herein for convenience only and is not to be taken as a limitation on the invention. For example, "upper", "lower", "front", "rear", "side", "longitudinal", "lateral", "transverse", "upwards", "downwards", "forward", "backward", "sideward", "left", "right", "horizontal", "vertical", "upward", "inner", "outer", "inward", "outward", "top", "bottom", "higher", "above", "below", "central", "middle", "intermediate", "between", "end", "adjacent", "proximate", "near", "distal", "remote", "radial", "circumferential", or the like, merely describe the configuration shown in the Figures. Indeed, the components may be oriented in any direction and the terminology, therefore, should be understood as encompassing such variations unless specified otherwise.

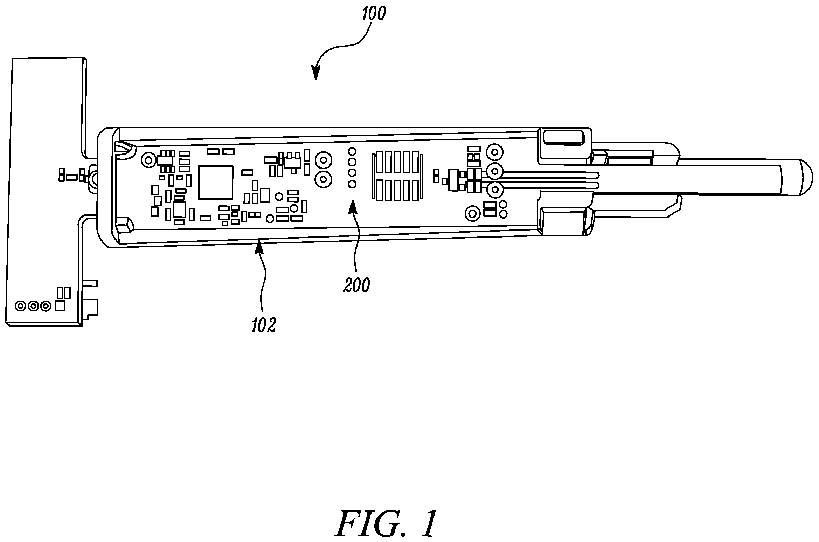

**FIG. 1** illustrates a schematic view of a soil moisture sensor **100.** The soil moisture sensor **100** is configured for measuring a moisture within a soil surrounding the soil moisture sensor **100.** The soil moisture sensor **100** is wirelessly connected with an irrigation system (not shown) to control irrigation. The soil moisture sensor **100** has a substantially T-shaped configuration. Further, the soil moisture sensor **100** comprises a PCB **200**, particularly a four-layer PCB **200**, also referred to as a printed circuit board **200.** The PCB **200** comprises electronic circuitry for determining an amount of soil moisture and/ or for transmitting results from the soil moisture measuring to a further component, e.g., the irrigation device, and/ or governing the functioning of the irrigation device. The PCB **200** is at least partially covered by a cover **102.**

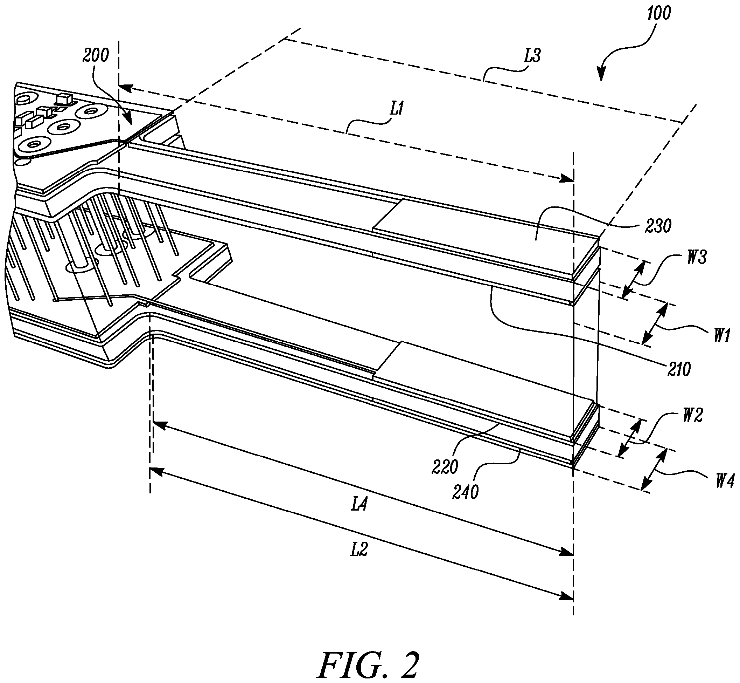

**FIG. 2** illustrates an exploded schematic view of the soil moisture sensor **100.** The soil moisture sensor **100** comprises the PCB **200** that further comprises a first conductive surface **210**, a second conductive surface **220,** a third conductive surface **230** and a fourth conductive surface **240.** The third conductive surface **230** is arranged adjacent to the first conductive surface **210.** Additionally, the first conductive surface **210** is arranged in-between the third conductive surface **230** and the second conductive surface **220.** In other words, the third conductive surface **230** and the second conductive surface **220** are arranged on opposite sides of the first conductive surface **210.** Further, the fourth conductive surface **240** is arranged adjacent to the second conductive surface **220.** Additionally, the second conductive surface **220** is arranged in-between the first conductive surface **210** and the fourth conductive surface **240.** In other words, the fourth conductive surface **240** and the first conductive surface **210** are arranged on opposite sides of the second conductive surface **220.**

Further, the first conductive surface **210,** the second conductive surface **220,** the third conductive surface **230** and the fourth conductive surface **240** each have an elongate structure. The first conductive surface **210** comprises a first length **L1** and a first width **W1** extending perpendicular to the first length **L1.** Further, the second conductive surface **220** comprises a second length **L2** and a second width **W2** extending perpendicular to the second length **L2.** Further, the third conductive surface **230** comprises a third length **L3** and a third width **W3** extending perpendicular to the third length **L3.** Further, the fourth conductive surface **240** comprises a fourth length **L4** and a fourth width **W4** extending perpendicular to the fourth length **L4.**

As illustrated in **FIG. 2****,** the first width **W1** and the second width **W2** are substantially equal, and the third width **W3** and the fourth width **W4** are substantially equal. At the same time, the first width **W1** is equal to the third width **W3,** and the second width **W2** is equal to the fourth width **W4.** Additionally, the first length **L1** is equal to the second length **L2,** and the third length **L3** is equal to the fourth length **L4.** For example, the first width **W1** of the first conductive surface **210,** the second width **W2** of the second conductive surface **220,** the third width **W3** of the third conductive surface **230** and the fourth width **W4** of the fourth conductive surface **240** have a respective width being substantially 3.5 mm. Further, for example, the first length **L1** of the first conductive surface **210,** the second length **L2** of the second conductive surface **220,** the third length **L3** of the third conductive surface **230** and the fourth length **L4** of the fourth conductive surface **240** have a respective length being substantially 12 mm.

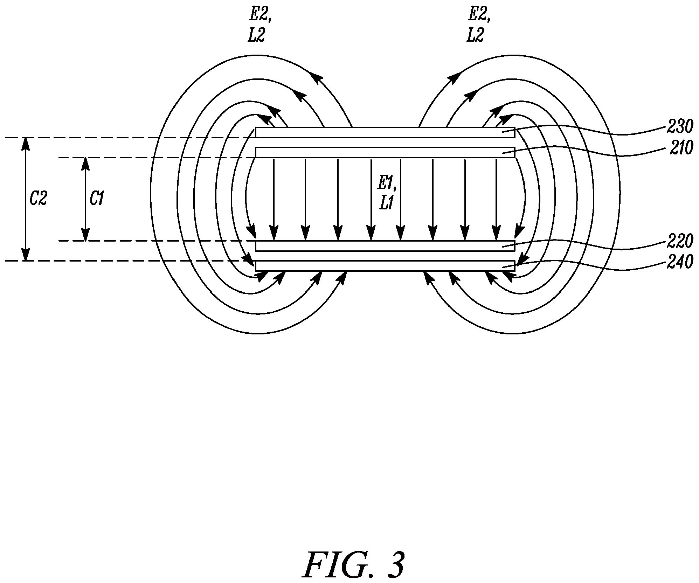

**FIG. 3** illustrates a schematic view of the first conductive surface **210,** the second conductive surface **220,** the third conductive surface **230** and the fourth conductive surface **240.** The first conductive surface **210** and the second conductive surface **220** form a first capacitor **C1.** The first capacitor **C1** is configured to generate a primary electric field **E1** between the first conductive surface **210** and the second conductive surface **220,** wherein the primary electric field comprises primary electric field lines **L1.** The primary electric field **E1** is a parasitic field.

Further, the third conductive surface **230** and the fourth conductive surface **240** form a second capacitor **C2.** The second capacitor **C2** is configured to generate a secondary electric field **E2** between the third conductive surface **230** and the fourth conductive surface **240,** wherein the secondary electric field **E2** comprises secondary electric field lines **L2.** The secondary electric field **E2** is a measuring field.

Further, the first capacitor **C1** and the second capacitor **C2** are arranged such that the first capacitor **C1** is nested within respectively between the second capacitor **C2.** Further, the primary electric field lines **L1** are separated from the secondary electric field lines **L1** such that a capacitance measuring of the moisture within the surrounding soil is enabled respectively allowed. In other words, the parasitic field is separated from the measuring field such that the capacitance measuring of the moisture within the surrounding soil is enabled respectively allowed.

As illustrated in FIG. 3, during operation of the soil moisture sensor **100,** the first capacitor **C1** generates the primary electrical field **E1** comprising a plurality of primary electrical field lines **L1** extending from the first conductive surface **210** to the second conductive surface **220.** The primary field lines **L1** are formed between the inner surfaces facing each other, and the side edges of the first conductive surface **210** and the second conductive surface **220.** The second capacitor **C2** generates the secondary electrical field **E2** comprising a plurality of secondary field lines **L2** extending from the third conductive surface **230** to the fourth conductive surface **240.** The secondary electrical field lines **L2** are formed between the outer surfaces facing away from each other, and the side edges of the third conductive surface **230** and the fourth conductive surface **240..**

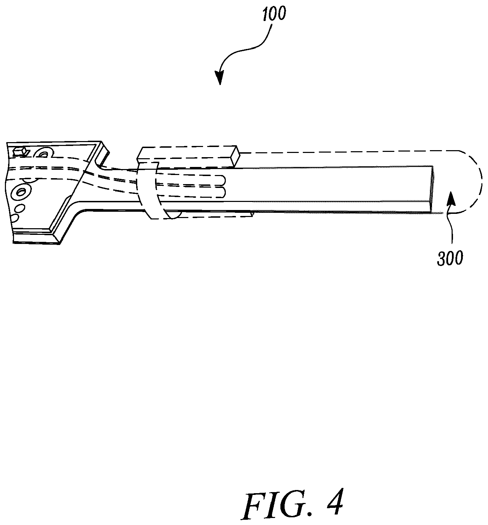

**FIG. 4** illustrates a schematic view of the soil moisture sensor **100** surrounded by a water-absorbing material **300.** At least the first conductive surface **210,** the second conductive surface **220,** the third conductive surface **230** and the fourth conductive surface **240** are surrounded by a water-absorbing material **300,** wherein the water-absorbing material **300** functions as an intermediary between the soil moisture sensor **100** and the surrounding soil.

In the drawings and specification, there have been disclosed exemplary embodiments and examples of the invention and, although specific terms are employed, they are used in a generic and descriptive sense only and not for the purpose of limitation of the scope of the invention being set forth in the following claims.

### LIST OF ELEMENTS

- **100**: Soil Moisture Sensor
- **102**: Cover
- **200**: PCB/ Printed Circuit Board
- **210**: First Conductive Surface
- **220**: Second Conductive Surface
- **230**: Third Conductive Surface
- **240**: Fourth Conductive Surface
- **300**: water-absorbing material
- **W1**: First Width
- **W2**: Second Width
- **W3**: Third Width
- **W4**: Fourth Width
- **L1**: First Length
- **L2**: Second Length
- **L3**: Third Length
- **L4**: Fourth Length
- **C1**: First Capacitor
- **C2**: Second Capacitor
- **E1**: Primary Electric Field
- **L1**: Primary Electric Field Lines
- **E2**: Secondary Electric Field
- **L2**: Secondary Electric Field Lines

## Claims

1. A soil moisture sensor (**100**) for measuring a moisture within a soil surrounding the soil moisture sensor (**100**), comprising:
a first capacitor (**C1**) configured to generate a primary electric field (E1) comprising primary electric field lines (**L1**),
**characterized in that:**
the soil moisture sensor (**100**) further comprises:
a second capacitor (**C2**) configured to generate a secondary electric field (**E2**) comprising secondary electric field lines (**L2**), and
wherein the first capacitor (**C1**) and the second capacitor (**C2**) are arranged such that the first capacitor (**C1**) is nested within the second capacitor (**C2**), and wherein the primary electric field lines (**L1**) are separated from the secondary field lines (**L2**) such that a capacitance measuring of the moisture within the surrounding soil is enabled.

2. The soil moisture sensor (**100**) according to claim 1,
wherein the first capacitor (**C1**) is formed by a first conductive surface (**210**) and second conductive surface (**220**), wherein the first conductive surface (**210**) and the second conductive surface (**220**) are configured such that the primary electric field (**E1**) is formed between the first conductive surface (**210**) and the second conductive surface (**220**),
wherein the second capacitor (**C2**) is formed by a third conductive surface (**230**) and a fourth conductive surface (**240**), wherein the third conductive surface (**230**) and the fourth conductive surface (**240**) are configured such that the secondary electric field (**E2**) is formed between the third conductive surface (**230**) and the fourth conductive surface (**240**),
wherein the first conductive surface (**210**) and the third conductive surface (**230**) are formed as two separate conductive surfaces, and
wherein the second conductive surface (**220**) and the fourth conductive surface (**240**) are formed as one common conductive surface.

3. The soil moisture sensor (**100**) according to claim 1,
wherein the first capacitor (**C1**) is formed by a first conductive surface (**210**) and a second conductive surface (**220**), wherein the first conductive surface (**210**) and the second conductive surface (**220**) are configured such that the primary electric field (**E1**) is formed between the first conductive surface (**210**) and the second conductive surface (**220**),
wherein the second capacitor (**C2**) is formed by a third conductive surface (**230**) and a fourth conductive surface (**240**), wherein the third conductive surface (**230**) and the fourth conductive surface (**240**) are configured such that the secondary electric field (**E2**) is formed between the third conductive surface (**230**) and the fourth conductive surface (**240**),
wherein the first conductive surface (**210**) and the third conductive surface (**230**) are formed as two separate conductive surfaces, and
wherein the second conductive surface (**220**) and the fourth conductive surface (**240**) are formed as two separate conductive surfaces.

4. The soil moisture sensor (**100**) according to any one of the preceding claims,
wherein the primary electric field (**E1**) is a parasitic field, and/or
wherein the secondary electric field (**E2**) is a measuring field.

5. The soil moisture sensor (**100**) according to any one of the preceding claims,
wherein the first capacitor (**C1**) and the second capacitor (**C1**) are simultaneously excited with a same signal

6. The soil moisture sensor (**100**) according to claim 5,
wherein the first capacitor (**C1**) and the second capacitor (**C2**) are excited with a 10kHz sinusoidal signal.

7. The soil moisture sensor (**100**) according to any one of the preceding claims,
wherein the capacitance measuring of the moisture within the surrounding soil is measured from the resulting current.

8. The soil moisture sensor (**100**) according to any one of the claims 2 to 7, wherein the third conductive surface (**230**) is arranged adjacent to the first conductive surface (**210**), and/or
wherein the first conductive surface (**210**) is arranged in-between the third conductive surface (**230**) and the second conductive surface (**220**).

9. The soil moisture sensor (**100**) according to any one of the claims 3 to 8, wherein the fourth conductive surface (**240**) is arranged adjacent to the second conductive surface (**220**), and/or
wherein the second conductive surface (**220**) is arranged in-between the first conductive surface (**210**) and the fourth conductive surface (**240**).

10. The soil moisture sensor (**100**) according to any one of the claims 2 to 9, wherein the first conductive surface (**210**) comprises a first width (**W1**) extending perpendicular to a first length (**L1**) of the first conductive surface (**210**),
wherein the second conductive surface (**220**) comprises a second width (**W2**) extending perpendicular to a second length (**L2**) of the second conductive surface (**220**),
wherein the third conductive surface (**230**) comprises a third width (**W3**) extending perpendicular to a third length (**L3**) of the third conductive surface (**230**), and
wherein the fourth conductive surface (**240**) comprises a fourth width (**W4**) extending perpendicular to a fourth length (**L4**) of the fourth conductive surface (**240**).

11. The soil moisture sensor (**100**) according to claim 10,
wherein the first width (**W1**) and the second width (**W2**) are substantially equal, and/or
wherein the third width (**W3**) and the fourth width (**W4**) are substantially equal, and/or
wherein the first length (**L1**) is substantially equal to the second length (**L2**), and/or
wherein the third length (**L3**) is substantially equal to the fourth length (**L4**).

12. The soil moisture sensor (**100**) according to claim 10 or claim 11, wherein the first width (**W1**) is greater than or substantially equal to the third width (**W3**), and/or
wherein the second width (**W2**) is greater than or substantially equal to the fourth width (**W4**).

13. The soil sensor (**100**) according to any one of the claims 10 to 12, wherein the first length (**L1**) is greater than or substantially equal to the third length (**L3**), and/or
wherein the second length (**L2**) is greater than or substantially equal to the fourth length (**L4**).

14. The soil moisture sensor (**100**) according to any of the preceding claims, wherein the soil moisture sensor (**100**) is at least partially surrounded by a water-absorbing material (3**0**0).

15. The soil moisture sensor (**100**) according to any of the preceding claims, wherein the soil moisture sensor (**100**) is wirelessly connected with an irrigation system to control irrigation.
